# EUROPÄISCHE PATENTSCHRIFT

(11) **EP 0 311 880 B1**
(45) Veröffentlichungstag und Bekanntmachung des Hinweises auf die Patenterteilung: **23.02.1994**
(21) Anmeldenummer: 88116480.0
(22) Anmeldetag: 05.10.1988
(51) Int. Cl.: C07C 33/035, C07C 29/09, C07C 41/30, C07C 41/20, C07C 45/29, C07C 29/32, C07C 67/08

(54) **1,9-Non-2-endiole, deren Abkömmlinge, sowie Verfahren zu ihrer Herstellung und Verwendung als Zwischenprodukte**
1,9-Non-2-endiols, their derivatives, processes for their preparation and use as intermediates
1,9 Non-2-endiols, leurs dérivés, ainsi que des procédés de fabrication et utilisation comme produits intermédiaires

(30) Priorität: 10.10.1987 DE 3734330
(43) Veröffentlichungstag der Anmeldung: 19.04.1989
(73) Patentinhaber: BASF Aktiengesellschaft, 67063 Ludwigshafen (DE)
(72) Erfinder: Brueckner, Christiane, Dr., D-6700 Ludwigshafen (DE); Buschmann, Ernst, Dr., D-6700 Ludwigshafen (DE); Mackenroth, Wolfgang, Dr., D-6702 Bad Duerkheim (DE); Becker, Rainer, Dr., D-6702 Bad Duerkheim (DE)

(56) Entgegenhaltungen:
- EP-A- 0 251 472
- THE JOURNAL OF THE CHEMICAL SOCIETY, PERKIN TRANSACTIONS I, 1982, Seiten 1333-1342, The Royal Society of Chemistry, Cambridge, GB; D.N. JONES et al.: "Synthesis of some thietanoprostanoids"

## Beschreibung

In 2-Stellung ungesättigte C₉-Verbindungen sind verschiedentlich als Zwischenprodukte beschrieben worden. Teils handelt es sich dabei um Acetylenalkohole und gewisse einfache Abkömmlinge, teils um olefinisch ungesättigte Verbindungen sowie deren Abkömmlinge, wie die nachstehende Übersicht zeigt.

### Bekannt:

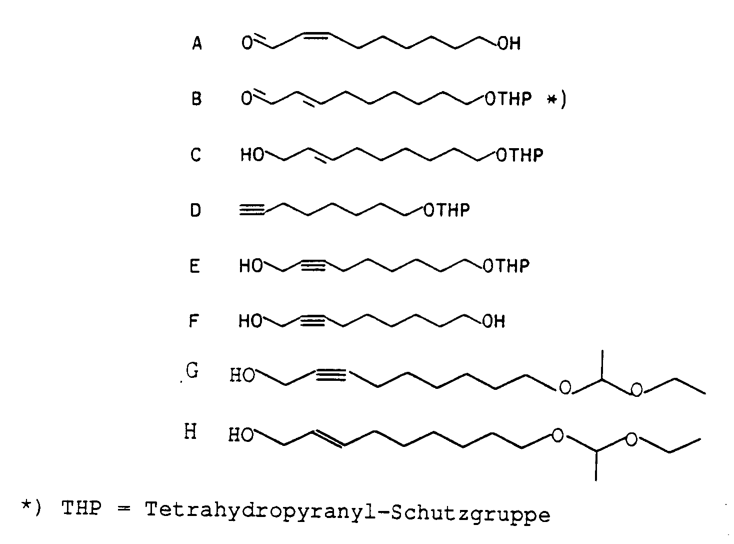

Diese Verbindungen und ihre Verwendung sind z.B. in folgenden Druckschriften beschrieben: J. Chem. Ecol. 8 (1), 195 (1982); Agric. Biol. Chem. 49 (1), 141 (1985); Chem. Ber. 100 (6), 1936 (1967); JACS 107 (7), 2124 (1985); J. Chem. Soc. PTI, 1982, 1333.

Eine an sich bekannte Verwendungsmöglichkeit für solche Verbindungen (auch mit anderer Kohlenstoffzahl als 9) ist der Aufbau längerkettiger Verbindungen mit einer oder mehreren Mehrfachbindungen an definierter Stelle. Verbindungen dieser Art spielen als Naturstoffe eine Rolle, besonders als Insektenlockstoffe (Pheromone) und es ist eine Aufgabe der Erfindung, zu solchen Lockstoffen einen günstigen, technisch nutzbaren Weg anzugeben.

Es wurde nun gefunden, daß 1,9-Non-2-endiol und Derivate dieses Diols der Formeln Ia und/oder Ib vorteilhaft als Zwischenprodukt zur Herstellung insbesondere von (E7,Z9)-Dodecadienylacetat verwendet werden können.

Unmittelbarer Erfindungsgegenstand sind 1,9-Non-2-endiolderivate der allgemeinen Formeln Ia und Ib, die zueinander im Verhältnis von cis- und trans-Isomeren stehen, nämlich 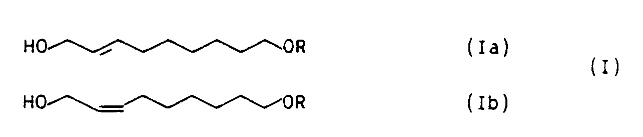
wobei R für Wasserstoff, einen C₁-C₆-Alkyl-, C₁-C₄-Acyl-, Tri-C₁-C₄-alkylsilyl-, Benzoyl- oder einen offenkettigen oder ringgeschlossenen C₁-C₆-Alkoxy-C₁-C₄-alkylrest, jedoch nicht Tetrahydropyranyl oder, im Fall von Ia, den 1-Ethoxy-ethylrest steht.

Cis- und trans-1,9-Non-2-endiol und deren in 9-Stellung durch eine abspaltbare Schutzgruppe verschlossene Abkömmlinge Ia bzw. Ib, ausgenommen die o.g. Verbindungen (C und H), sind neue Verbindungen.
Der Rest R bedeutet vorzugsweise
- eine Alkylgruppe mit 1 bis 6, insbesondere 1 bis 4 Kohlenstoffatomen wie Methyl, Ethyl, Propyl, 1-Methylethyl, Butyl, 1-Methylpropyl, 2-Methoxypropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl und 1,1-Dimethylethyl,
- eine Acylgruppen mit 1 bis 4 Kohlenstoffatomen wie Methylcarboxy, Ethylcarboxy, Propylcarboxy, i-Propylcarboxy,
- eine Trialkylsilylgruppen mit 1 bis 4 Kohlenstoffatomen je Alkylgruppe wie Trimethylsilyl, Ethyldimethylsilyl, Diethylmethylsilyl, Triethylsilyl, Propyldimethylsilyl, i-Propyldimethylsilyl und tert.-Butyldimethylsilyl, insbesondere Trimethylsilyl und tert.-Butyldimethylsilyl,
- eine Alkoxyalkylgruppe mit insgesamt 1 bis 10, insbesondere 1 bis 4 Kohlenstoffatomen wie Methoxymethyl, Methoxyethyl, Ethoxymethyl und Ethoxyethyl,
- einen ringgeschlossenen Alkylether wie Tetrahydrofuranyl, jedoch nicht Tetrahydropyranyl
sowie Wasserstoff oder eine Benzoylgruppe.

Insbesondere die tert.-Butyl geschützten Verbindungen sind wertvoll als Zwischenprodukte, mit deren Hilfe vor allem das Pheromon des bekreuzten Traubenwicklers (Lobesia botrana) auf einem neuen Weg einfach und mit hoher Ausbeute in technischem Maßstab herstellbar ist.

Dieser Wirkstoff wurde 1973 erstmals beschrieben (Mitteilungen der schweizerischen entomologischen Gesellschaft 46, 71-73 (1973), US-Patent 3 845 108, DOS-24 40 759). Das dort ebenfalls beschriebene Herstellverfahren weist 10 meist aufwendige Verfahrensschritte auf und ist somit sehr umständlich; zur Synthese großer Mengen, wie sie zur großflächigen Verwendung von Pheromonwirkstoffen zur Insektenkontrolle mittels der Konfusionsmethode erforderlich sind, ist es nicht geeignet. Wegen der Vorteile der biologischen Schädlingsbekämpfung mit Pheromonen (sehr spezifische, nützlingsschonende Wirkstoffe, keine Resistenzerscheinungen, gute biologische Abbaubarkeit, außerordentlich geringe Toxizität) ist eine in technischem Maßstab wirtschaftliche Synthese dringend gesucht.

Die erfindungsgemäßen Non-2-endiol-Verbindungen der Formel Ia und Ib erhält man leicht durch Umsetzung der bekannten Acetylenderivate des Typs II (J. Med. Chem. 10, 533 (1967)) mit Formaldehyd in Gegenwart oder nach Einwirkung einer starken Base wie beispielsweise eines Lithiumorganyls oder einer Grignard-Verbindung (n-Buli, EtMgHal, MeMgHal) wie üblich bei einer Temperatur von bis zu -20°C in einem geeigneten aprotischen Lösungsmittel wie z. B. Tetrahydrofuran oder Diethylether und anschließende Hydrierung. Formaldehyd wird vorteilhaft als handelsüblicher Paraformaldehyd eingesetzt.

Eine Möglichkeit, unter Verwendung der erfindungsgemäß als Schlüsselprodukt dienenden Verbindung I zu den gesuchten Naturstoffen zu kommen, ergibt sich aus dem nachstehenden Schema, wobei für R stets der t-Butylrest gewählt wurde:

### Schema:

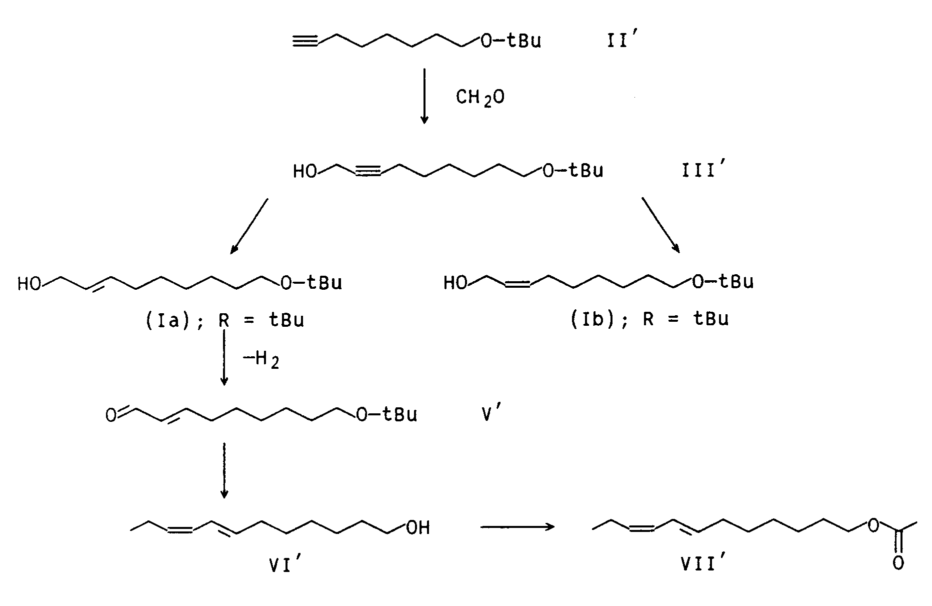

Aus den Verbindungen Ia und Ib lassen sich durch Oxidation die entsprechenden 1-Oxo-Verbindungen V nämlich Aldehydalkohole bzw. Aldehydether gewinnen, die mittels Wittig-Reaktion unter entsprechender Kettenverlängerung in die unmittelbaren Vorprodukte der gesuchten Naturstoffe, d.h. Dienole VI überführt und ggf. dann verestert werden können VII (GB-Patent 2 098 609 bzw. Liebigs Ann. Chem. 1981, 1705).

Eine praktische Ausgestaltung der im vorstehenden Schema wiedergegebenen Umsetzungsfolge ist in den nachfolgenden Beispielen gezeigt.

### Beispiel

1. Zu einer Lösung von 182 g (1,00 mol) 8-tert.-Butoxyoctin in 1 l THF werden 1,20 mol n-Buli in Hexan bei Raumtemperatur zugetropft und 1 Stunde nachgerührt. Die so erhaltene Lösung wird bei -30 bis -40°C langsam zu 180 g (6,00 mol) Paraformaldehyd in 1 l THF zugegeben und 1 Stunde bei -30°C sowie weitere 20 Stunden unter Aufwärmen auf Raumtemperatur gerührt. Anschließend wird das Reaktionsgemisch auf Eiswasser gegossen und 3mal mit Methyl-tert.-butylether extrahiert. Nach Trocknen und Einengen erhält man 214 g Produkt, das nach gaschromatographischer Untersuchung einen Gehalt von 73 % 9-tert.-Butoxynon-2-in-1-ol (entsprechend 74 % Ausbeute) aufweist.
2. Das erhaltene Noninol, bzw. dessen Derivate können je nach den gewählten Reduktionsbedingungen in Z- bzw. E-Nonenole umgewandelt werden:
   a) 130 g (0,610 mol) 9-tert.-Butoxynon-2-in-1-ol in 1 l Methanol nehmen unter Einwirkung von 5 g Lindlar-Katalysator bei 20 bis 30°C in 1,5 Stunden 11 l Wasserstoff auf. Nach Abfiltrieren und Einengen erhält man 128 g Produkt, das nach gaschromatographischer Untersuchung zu 81 % aus (Z)-tert.-Butoxynon-2-en-1-ol (entsprechend 80 % der rechnerisch möglichen Ausbeute) besteht.
   b) Zu einer Lösung von 25,0 g (0,650 mol) Lithiumaluminiumhydrid in 800 ml THF werden 34,0 g (0,215 mol) 2-Nonin-1,9-diol in 200 ml THF bei 20 bis 30°C zugetropft und 20 h bei Raumtemperatur gerührt. Nach anschließendem zweistündigen Erhitzen unter Rückfluß erfolgt wäßrige Aufarbeitung durch Zugabe von gesättigter Ammoniumchlorid-Lösung. Dreimalige Extraktion mit Methyl-tert.-butylether, Waschen mit NH₄Cl-Lösung, Trocknen und Einengen liefern 33,0 g Produkt, das nach gaschromatographischer Untersuchung zu 94 % aus (E)-2-Nonen-1,9-diol (entsprechend 91 % der rechnerisch möglichen Ausbeute) besteht.
3. Zu einer Lösung von 107 g (0,500 mol) (Z)-tert.-Butoxynon-2-en-1-ol in 1 l Diethylether werden 200 g (2,30 mol) Mangandioxid zugegeben und 20 Stunden bei Raumtemperatur nachgerührt. Nach Abfiltrieren wird 2mal mit je 500 ml 2N HCl sowie 2mal mit je 500 ml 10 %iger NaHCO₃-Lösung gewaschen. Trocknen und Einengen liefern 104 g Produkt, das nach gaschromatographischer Untersuchung einen Gehalt von 92 % (Z)-tert.-Butoxynon-2-en-1-al (entsprechend 90 % der rechnerisch möglichen Ausbeute) aufweist.

## Patentansprüche (Patentansprüche für folgende(n) Vertragsstaat(en): CH, DE, FR, GB, IT, LI, NL)

1. 1,9-Non-2-endiol sowie dessen in 9-Stellung eine abspaltbare Schutzgruppe tragende Abkömmlinge der allgemeinen Formeln Ia und Ib 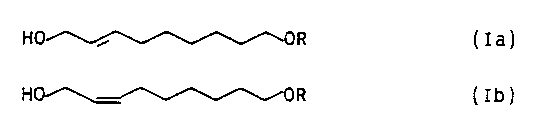 wobei R für Wasserstoff, einen C₁-C₆-Alkyl-, C₁-C₄-Acyl-, Tri-C₁-C₄-alkylsilyl-, Benzoyl- oder einen offenkettigen oder ringgeschlossenen C₁-C₆-Alkoxy-C₁-C₄-alkylrest, jedoch nicht den Tetrahydropyranylrest oder, im Fall von Ia, den 1-Ethoxyethylrest steht.

2. Verfahren zur Herstellung einer Verbindung der Formel Ia und Ib gemäß Anspruch 1, dadurch gekennzeichnet, daß man den entsprechenden Oktinolether II 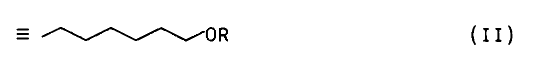 in Gegenwart einer Base in einem inerten organischen Lösungsmittel in an sich bekannter Weise mit Formaldehyd zum Derivat III 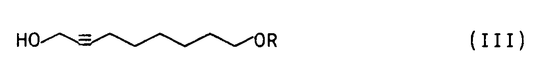 kondensiert, zum Nonenol Ia und Ib reduziert und gegebenenfalls hydrolysiert (Ia und Ib, R=H).

3. Verfahren zur Herstellung von physiologisch wirksamem (E7,Z9)-Dodecadienol bzw. dessen Essigsäureester, dadurch gekennzeichnet, daß man eine Verbindung der Formel Ia und/oder Ib gemäß Anspruch 1 zum Aldehydether V 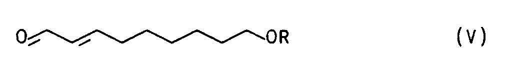 oxidiert, diesen durch Wittig-Reaktion in das Dodekadienol VI 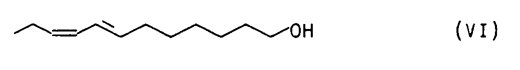 überführt und ggf. verestert zu VII 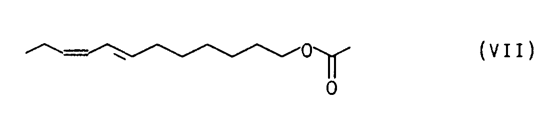 und/oder eine Abtrennung des jeweils unerwünschten Stereoisomeren vornimmt.

## Patentansprüche (Patentansprüche für folgende(n) Vertragsstaat(en): ES)

1. Verfahren zur Herstellung von 1,9-Non-2-endiol sowie dessen in 9-Stellung eine abspaltbare Schutzgruppe tragenden Abkömmlingen der allgemeinen Formeln Ia und Ib 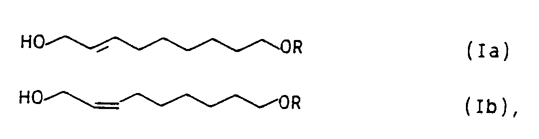 wobei R für Wasserstoff, einen C₁-C₆-Alkyl-, C₁-C₄-Acyl-, Tri-C₁-C₄-alkylsilyl-, Benzoyl- oder einen offenkettigen oder ringgeschlossenen C₁-C₆-Alkoxy-C₁-C₄-alkylrest, jedoch nicht den Tetrahydropyranylrest oder, im Fall von Ia, den 1-Ethoxyethylrest steht, dadurch gekennzeichnet, daß man den entsprechenden Oktinolether II 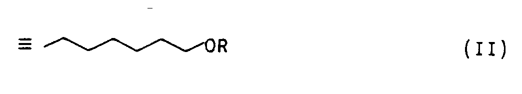 in Gegenwart einer Base in einem inerten organischen Lösungsmittel in an sich bekannter Weise mit Formaldehyd zum Derivat III 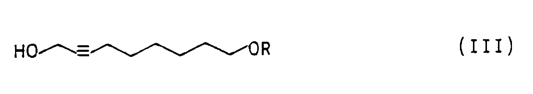 kondensiert, zum Nonenol Ia und Ib reduziert und gegebenenfalls hydrolysiert (Ia und Ib, R=H).

2. Verfahren zur Herstellung von physiologisch wirksamem (E7,Z9)-Dodecadienol bzw. dessen Essigsäureester, dadurch gekennzeichnet, daß man eine Verbindung der Formel Ia und/oder Ib gemäß Anspruch 1 zum Aldehydether V 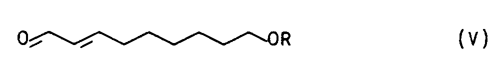 oxidiert, diesen durch eine Wittig-Reaktion in das Dodekadienol VI 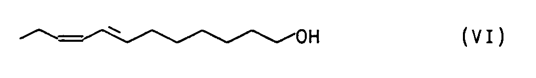 überführt und ggf. verestert zu VII 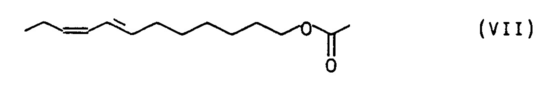 und/oder eine Abtrennung des jeweils unerwünschten Stereoisomeren vornimmt.

## Claims (Claims for the following Contracting State(s): CH, DE, FR, GB, IT, LI, NL)

1. 1,9-Non-2-enediol and its derivatives which carry in the 9-position a protective group which can be eliminated, of the formula Ia and Ib 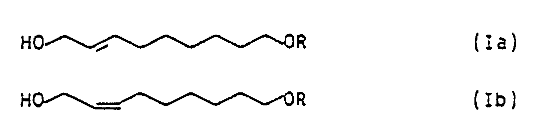 where R is hydrogen, C₁-C₆-alkyl, C₁-C₄-acyl, tri-C₁-C₄-alkylsilyl, benzoyl or an open-chain or cyclic C₁-C₆-alkoxy-C₁-C₄-alkyl radical, but not tetrahydropyranyl or, in the case of Ia, 1-ethoxyethyl.

2. A process for the preparation of a compound of the formula Ia or Ib as claimed in claim 1, wherein an appropriate octynol ether II 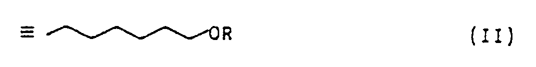 is condensed with formaldehyde in the presence of a base in an inert organic solvent in a conventional manner to give a derivative III 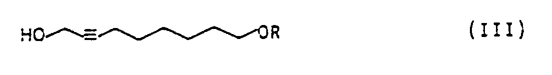 the latter is reduced to a nonenol Ia or Ib, which may be hydrolyzed (Ia or Ib, where R is H).

3. A process for the preparation of physiologically active (E7,Z9)-dodecadienol and its acetate, wherein a compound of the formula Ia and/or Ib as claimed in claim 1 is or are oxidized to an aldehyde ether V 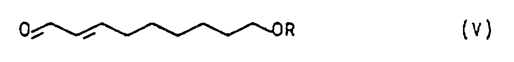 the latter is converted by a Wittig reaction into a dodecadienol VI 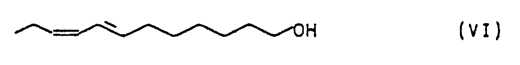 which may be esterified to VII 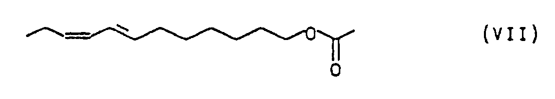 and/or the particular stereoisomer not desired is separated off.

## Claims (Claims for the following Contracting State(s): ES)

1. A process for the preparation of 1,9-Non-2-enediol and its derivatives which carry in the 9-position a protective group which can be eliminated, of the formulae Ia and Ib 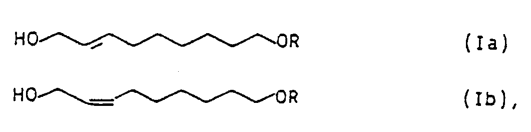 where R is hydrogen, C₁-C₆-alkyl, C₁-C₄-acyl, tri-C₁-C₄-alkylsilyl, benzoyl or an open-chain or cyclic C₁-C₆-alkoxy-C₁-C₄-alkyl radical, but not tetrahydropyranyl or, in the case of Ia, 1-ethoxyethyl, wherein an appropriate octynol ether II 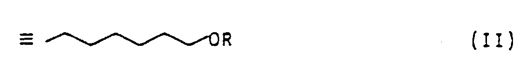 is condensed with formaldehyde in the presence of a base in an inert organic solvent in a conventional manner to give a derivative III 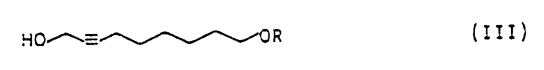 the latter is reduced to a nonenol Ia or Ib, which may be hydrolyzed (Ia or Ib, where R is H).

2. A process for the preparation of physiologically active (E7,Z9)-dodecadienol or its acetate, wherein a compound of the formula Ia and/or Ib as claimed in claim 1 is or are oxidized to an aldehyde ether V 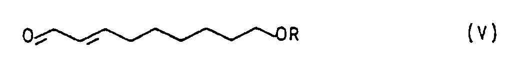 the latter is converted by a Wittig reaction into the dodecadienol VI 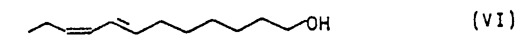 which may be esterified to VII 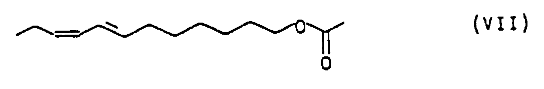 and/or the particular stereoisomer not desired is separated off.

## Revendications (Revendications pour l'(les) Etat(s) contractant(s) suivant(s): CH, DE, FR, GB, IT, LI, NL)

1. 1,9-non-2-en diol ainsi que ses dérivés portant en position 9 un groupe protecteur éliminable, de formules générales Ia et Ib 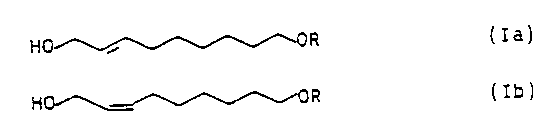 R étant mis pour hydrogène, un reste alkyle en C1-C6, acyle en C1-C4, tri-alkyle en C1-C4-silyle, benzoyle ou un reste alcoxy en C1-C6-alkyle en C1-C4 à chaîne ouverte ou fermée en cycle, mais toutefois pas le reste tétrahydropyranyle ou, dans le cas de la, le reste 1-éthoxyéthyle.

2. Procédé de préparation d'un composé de formule Ia et Ib selon la revendication 1, caractérisé par le fait que l'on condense, de manière connue en soi, l'octynoléther II 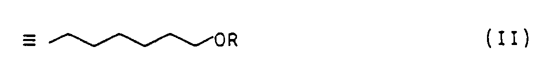 en dérivé III 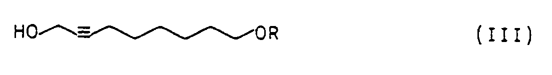 avec du formaldéhyde en présence d'une base dans un solvant organique, on le réduit en nonenol Ia et Ib et on l'hydrolyse éventuellement (Ia et Ib, R = H)

3. Procédé de préparation de (E7, Z9)-dodécadiénol ou ses esters acétiques, efficaces physiologiquement, caractérisé par le fait qu'on oxyde un composé de formule la et/ou Ib selon la revendication 1, en aldéhydéther V 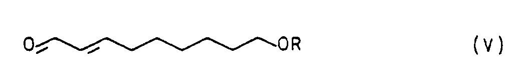 on transforme celui-ci par réaction de Wittig en le dodécadiénol VI 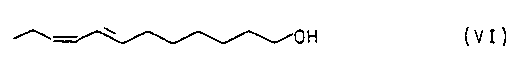 et on l'estérifie éventuellement en VII 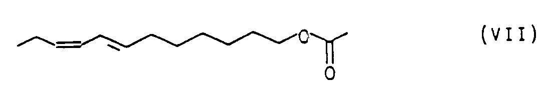 et/ou on procède à une séparation du stéréoisomère indésirable.

## Revendications (Revendications pour l'(les) Etat(s) contractant(s) suivant(s): ES)

1. Procédé de préparation de 1,9-non-2-en diol ainsi que ses dérivés portant en position 9 un groupe protecteur éliminable, de formules générales Ia et Ib 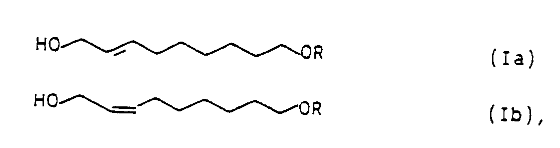 R étant mis pour hydrogène, un reste alkyle en C1-C6, acyle en C1-C4, trialkyle en C1-C4-silyle, benzoyle ou un reste alcoxy en C1-C6-alkyle en C1-C4 à chaîne ouverte ou fermée en cycle, mais toutefois pas le reste tétrahydropyranyle ou, dans le cas de la, le reste 1-éthoxyéthyle, caractérisé par le fait que l'on condense, de manière connue en soi, l'octynoléther II 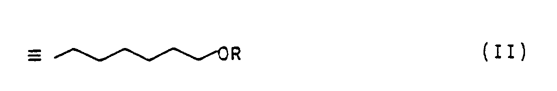 en dérivé III 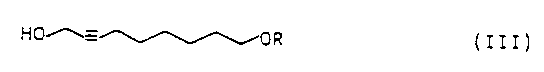 avec du formaldéhyde en présence d'une base dans un solvant organique, on le réduit en nonenol Ia et Ib et on l'hydrolyse éventuellement (Ia et Ib, R = H).

2. Procédé de préparation de (E7, Z9)-dodécadiénol ou ses esters acétiques, efficaces physiologiquement, caractérisé par le fait qu'on oxyde un composé de formule Ia et/ou Ib selon la revendication 1, en aldéhydéther V 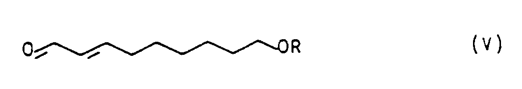 on transforme celui-ci par réaction de Wittig en le dodécadiénol VI 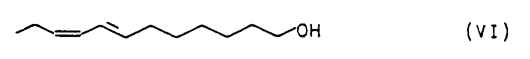 et on l'estérifie éventuellement en VII 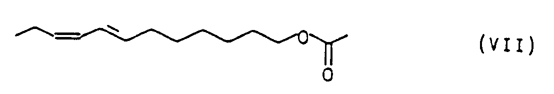 et/ou on procède à une séparation du stéréoisomère indésirable.
